Europäisches Patentamt

(19) European Patent Office

Office européen des brevets

(11) Publication number: 0 186 632
A2

# (12) EUROPEAN PATENT APPLICATION

(21) Application number: 85830309.2

(22) Date of filing: 18.12.85

(51) Int. Cl.⁴: A 61 F 2/10

(30) Priority: 27.12.84 IT 2426584

(43) Date of publication of application:
02.07.86 Bulletin 86/27

(84) Designated Contracting States:
AT BE CH DE FR GB LI LU NL SE

(71) Applicant: C.IT.I.C. S.r.l. Centro Italiano di Implantologia
di Capelli Artificiali
Borgo Pio 91
I-00193 Roma(IT)

(72) Inventor: Onorati, Giuseppe
Viale Piave 22
Milano(IT)

(72) Inventor: Forte, Mauro
Via S. Michele del Carso 2
Milano(IT)

(74) Representative: Rapisardi, Mariacristina, Dr. Proc.
Largo V Alpini 15
I-20145 Milano(IT)

(54) An artificial implantological hair and a needle for its insertion into the subcutaneous.

(57) The present invention relates to an artificial implantolo-gical hair which comprises in a portion thereof a knot self-tying during its sliding movement along the fiber of the artificial hair. The cited knot once tied on the artificial hair fiber has a bulk dimension substantially equal to the diameter dimension of the artificial hair itself, and a lug defining with the end portion of the artificial hair a hooked configuration which cooperates with the knot to retain the artificial hair within the subcutaneous. For inserting the artificial hair into the subcutaneous, a device is provided which cooperates with a body wherein a needle is slidable which has sufficient hardness to penetrate the subcutaneous and high elasticity effective to enable it to move back through the same path opened in the subcutaneous during the introduction of the artificial hair.

FIG. 1

EP 0 186 632 A2

AN ARTIFICIAL IMPLANTOLOGICAL HAIR AND A NEEDLE FOR
ITS INSERTION INTO THE SUBCUTANEOUS.

DESCRIPTION

The present invention relates to an artificial implantological hair and a needle for its insertion into the subcutaneous.

As is known, as the result of clinic experimentation, artificial hair have been developed in recent years which are elastic, well tolerated, non-toxic, and resistant to physical and chemical agents.

In general, nearly all such artificial hair have a loop end which can be engaged by the tip of a needle to implant the hair into the subcutaneous, said tip being made as thin as possible to avoid damaging the patient's skin.

Implantological artificial hair according to the prior art are currently formed, in general, from a polyester yielded by a reaction with polyethylene terephtalate and ethylene glycol so as to induce no immuno logical reactions in the recipient, on account of polyester being an inert material.

Such prior hair have, in accordance with the various techniques deve loped in recent years, a loop end which, additionally to being engagea ble by a needle tip for implantation into the subcutaneous, characteri stically ensures that the hair is retained securely within the subcuta neous.

One of the techniques employed to form that loop provides for the loop to be defined by an end portion of the hair bent over and adhered to the hair itself by microwave welding, thereby a closed loop is obtained having a jutting extremity at an angle of about 50 degrees from the hair; thus, a barb or hook is configured within the subcutaneous which tends to resist any force directed to pull the artificial hair out of the subcutaneous.

That technique has the drawback that a deposit of granulate occurs over the weldments on the hair fiber which originates from changes taking place in the fiber itself during the microwave welding process.

The above granulate, additionally to becoming a germ nesting area, has the disadvantage of causing in the epithelial tissue a contused lacerate wound which is the cause for subcutaneous irritation leading to possible acute and chronic infections which hinder re-epithelisation even 20 to 25 days after the artificial hair has been implanted.

Furthermore, that welding method tends to be weak and fragile, in that the loop may be broken during the hair implantation, thereby, after some time, it tends to slip off the skin since it no longer affords any adequate amount of drag.

It is in view of the foregoing factors that it is statistically reported that 20% to 25% of the hair implanted in the subcutaneous are lost on per annum basis.

According to a Japanese technique, the loop is formed so that a slipknot loop is provided whic is bulkier than the diameter of the artificial hair fiber, thereby on implanting the artificial hair into the skin a contuse lacerated wound is caused having a larger diameter than the hair fiber.

A wound of such a size favours bacterial nesting and results, accordingly, in abnormal inflammatory reactions which hinder even re-epithelisation of the skin and favour ejection of the hair from the subcutaneous after some time.

It is the aim of this invention to overcome such prior problems by providing an artificial implantological hair and a needle for its insertion into the subcutaneous which can produce, as the hair is being driven into the subcutaneous, a linear wound in the skin permitting even re-epithelisation of the skin and, hence, preventing bacterial nestings which over a time would result in the hair being rejected.

Within the above aim, it is an important object of the invention to provide an artificial implantological hair which can have, once driven into the subcutaneous, a high tensile strength.

Another object of the invention is to provide an artificial implantological hair the rate of exit whereof over one year can be as low as 4% to 5%.

A further object of the invention is to provide an artificial implatological hair which can ensure complete immovability of the same in the subcutaneous without lacerating or injuring the surrounding tissue and forming infection.

A not least object of the invention is to provide an artificial implatological hair which, by being adapted to be implanted in the subcutaneous to a shallow depth, can ensure first intention healing, i.e.

original reconstruction of the tissue.

An additional object of the invention, for another aspect thereof, is to provide a needle for inserting an artificial hair into the subcutaneous which is adapted to be drawn back through the same path opened in the subcutaneous during the artificial hair implantation process.

The above aim, and these and other objects to become apparent herein below,, are achieved by an artificial implantological hair characterised in that it comprises in a first portion thereof a knot adapted to self-tie while sliding along the artificial hair fiber, said knot as tied on said fiber having bulk dimensions substantially equal to the diameter of said artificial hair and a lug defining in conjunction with an end portion of said artificial hair a hooked configuration.

According to another aspect of this invention, the above aim, as well as these and other objects, are achieved by a needle for inserting an artificial hair into the subcutaneous, characterised in that it has a sufficient hardness for penetration of the subcutaneous and a high elasticity effective to enable it to be drawn back through the same path opened in said subcutaneous.

Further features and advantages of the invention will be apparent from the following description of a preferred but not exclusive embodiment of the inventive artificial implatological hair and needle for its insertion into the subcutaneous, with

- 5 -

0186632

reference to the accompanying illustrative and not limitative drawing, where:

Figure 1 shows the implementation of the self-tying slipknot according to the invention;

Figure 2 shows the needle tip in engagement with the knot of Figure 1 according to the invention;

Figure 3 shows the needle engaged with the hair during introduction into the subcutaneous, according to the invention;

Figure 4 shows the hair inserted in the subcutaneous prior to withdrawal of the needle according to the invention;

Figure 5 shows the hair in its inserted condition in the subcutaneous, according to the invention; and

Figure 6 is a perspective view showing a device incorporating the needle for introducing the artificial hair into the subcutaneous, according to the invention.

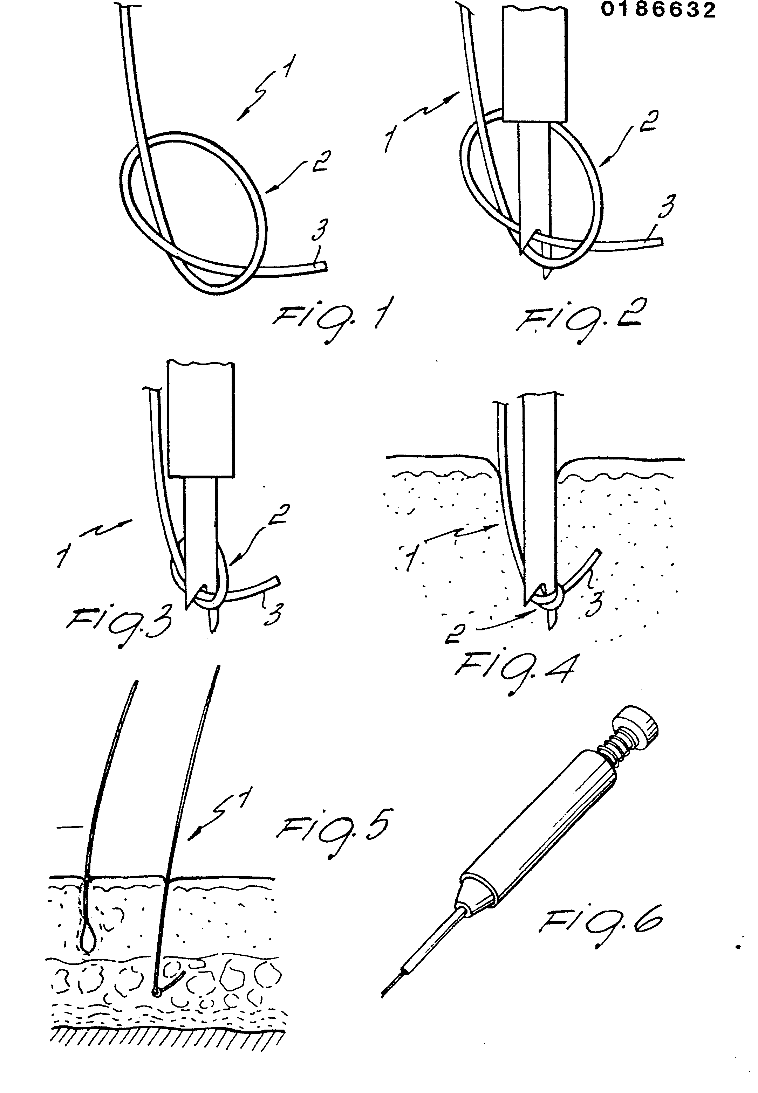

With particular reference to Figure 1, the artificial hair of this invention, comprehensively designated 1, comprises in a portion thereof near one of its ends a not, indicated at 2, which is usually termed a surgical knot and features self-tying properties on sliding along the artificial hair fiber to move to such a position as to allow a lug 3 to jut out of it which defines in cooperation with the artificial hair itself a hooked configuration adapted to engage under the skin.

As may be seen from Figure 5, the artificial hair will be retained in the subcutaneous by the knot 2 provided which practically forms a small ball and by the lug 3 which by cooperating with the latter secures the artificial hair to the skin and affords a loss rate of the latter as low as 4% or 5% over one year.

It should be further pointed out that with this type of knot, being the subject of this invention, full stability of the artificial hair is only achieved eight days after it has been driven into the subcutaneous, that is on conclusion of the inflammatory process which, as clinically tested, is a very brief one with respect to that brought about by the prior art techniques whereby full stability is achieved after 20 to 25 days owing to skin re-epithelisation being difficult to obtain.

It should be also pointed out that the needle for introducing the artificial hair into the subcutaneous is formed from 18/8 stainless steel so as to have advantageously sufficient hardness to perforate the subcutaneous and at the same time high elasticity to be less traumatic fro the wound and traverse always backwardly the same path it traversed during the insertion into the subcutaneous, contrary to prior needles which on meeting any generic resistance within the subcutaneous are unable, on withdrawal, to move through the same path along which they entered the subcutaneous and may, therefore, originate a contused lacerate wound which has all the contraindications mentioned above.

Merely by way of example, it should be added that conventional needles, once bent, must be discarded whereas the needle of this invention can be straightened up.

It has been found in actual practice that the device of this invention is particularly advantageous in ensuring a clinical and aesthetical prognosis which is definitely superior to those obtained with traditional methods.

The invention herein is susceptible to many modifications and changes without departing from the scope of the inventive concept; furthermore, all the details may be replaced with technical equivalents thereof.

## CLAIMS

1. An artificial implatological hair, characterised in that it comprises in a first portion thereof a knot adapted to self-tie while sliding along the artificial hair fiber, said knot as tied on said fiber having bulk dimensions substantially equal to the diameter dimension of said artificial hair and a lug defining in conjunction with an end portion of said artificial hair a hooked configuration.

2. A needle for inserting an artificial hair into the subcutaneous, characterised in that it has sufficient hardness for penetration of the subcutaneous and high elasticity effective to enable it to be drawn back through the same path opened in said subcutaneous.

3. A needle according to Claim 2, characterised in that it is formed from 18/8 stainless steel.

4. A needle according to Claims 2 and 3, characterised in that it has substantially the same diameter as said fiber and said knot.

5. An artificial implatological hair and needle for the insertion thereof into the subcutaneous, characterised in that they comprise one or more of the features herein described and/or illustrated.

All the above substantially as herein described, illustrated,-claimed, and for the objects set forth.

- 1/1 -

0186632

Fig. 1

Fig. 2

Fig. 3

Fig. 4

Fig. 5

Fig. 6